# FASCICULE DE BREVET EUROPEEN

(11) **EP 0 362 765 B1**
(45) Date de publication et mention de la délivrance du brevet: **15.09.1993**
(21) Numéro de dépôt: 89118231.3
(22) Date de dépôt: 02.10.1989
(51) Int. Cl.: A61B 3/00

(54) **Appareil pour l'observation et/ou le traitement d'un patient assis ou couché**
Vorrichtung zur Beobachtung und/oder Behandlung eines sitzenden oder liegenden Patienten
Apparatus for the observation and/or the treatment of a patient, seated or lying

(30) Priorité: 06.10.1988 CH 3747/88; 10.10.1988 FR 8813399
(43) Date de publication de la demande: 11.04.1990
(73) Titulaire: HAAG-STREIT AG, CH-3098 Köniz (CH)
(72) Inventeur: Gisel, Heinz, CH-8810 Horgen (CH); Bätscher, Paul, CH-3110 Münsingen (CH)
(74) Mandataire: de Raemy, Jacques

(56) Documents cités:
- EP-A- 0 209 992
- GB-A- 1 162 558
- US-A- 3 201 795
- US-A- 4 643 547

## Description

La présente invention se rapporte au domaine du matériel médical. Plus particulièrement, la présente invention à trait à un appareil pour l'observation et/ou le traitement de l'oeil d'un patient.

Depuis l'avènement et l'essor de la chirurgie ophtalmologique, les appareils utilisés n'ont cessé de progresser en précision et en performance (grâce aux applications du laser, notamment, voir par éxample EP-A-209 992).

Néanmoins, il était jusqu'à présent impossible d'utiliser le même appareil pour réaliser l'examen et/ou le traitement sur des patients assis et sur des patients couchés.

Un tel inconvénient faisait que l'équipe médicale était soit contrainte d'investir dans des appareils spécifiques pour chaque position, soit de ne travailler que dans certaines conditions limitées (difficulté de traiter un patient anesthésié en position assise, par exemple).

En tout état de cause, une telle lacune alourdissait les charges de l'équipe médicale ou bien limitait ses activités.

La présente invention a pour but de pallier cette lacune, en proposant un appareil pour la chirurgie opthalmologique par laser.

A cet effet, l'appareil selon l'invention, pour l'observation et le traitement d'un patient, comporte un dispositif d'observation et de traitement définissant un axe optique de sortie, un équipage pour assurer le positionnement et le réglage du dispositif, un châssis pour supporter l'équipage et le dispositif, ainsi qu'un système d'appui-tête pour caler et positionner la tête du patient. Cet appareil est caractérisé en ce que l'équipage comprend un support monté rigidement sur le châssis, une armature solidaire du dispositif d'observation et de traitement et une articulation qui relie le support et l'armature, agencée pour permettre une rotation de l'armature autour d'un axe horizontal, de manière à assurer une orientation angulaire de l'axe optique selon deux directions permettant respectivement l'observation et/ou le traitement d'un patient en positions assise et couchée.

Grâce à ces caractéristiques essentielles, l'appareil selon l'invention permet de soigner indifféremment les patients en position assise ou couchée.

De plus, comme cela apparaîtra dans la description qui va suivre, le médecin n'a besoin de changer ni de place, ni de position selon qu'il soigne un patient assis ou couché. Il peut rester assis au même endroit quelle que soit la position du patient.

D'autres caractéristiques et avantages de l'appareil selon la présente invention, apparaîtront à la lecture de la description détaillée qui va suivre faite à titre d'exemple non limitatif et se référant aux dessins annexés dans lesquels :
- la figure 1 est une représentation schématique d'un appareil selon l'invention vu de profil, utilisé sur un patient placé en position assise;
- la figure 2 est la représentation schématique du même appareil vu de face, dans les mêmes conditions d'utilisations;
- la figure 3 est la représentation schématique du même appareil, vu de profil utilisé sur un patient en position allongée;
- la figure 4 est la représentation du même appareil vu de face, dans des conditions d'utilisation identiques à celles de la figure 3;
- la figure 5 est la représentation en coupe des moyens pour caler la tête du patient en position allongée;
- la figure 6 est la représentation schématique de la vue en perspective de l'appareil représenté aux figures 1 à 4, dans les conditions d'utilisation identiques à celles de la figure 3;
- la figure 7 est la représentation schématique d'une partie des moyens de support, quand l'appareil est utilisé sur un patient assis;
- la figure 8 est la représentation schématique des mêmes moyens de support, quand l'appareil est utilisé sur un patient allongé; et
- la figure 9 est la représentation schématique des mêmes moyens de support, vus de face, quand l'appareil est utilisé sur un patient assis.

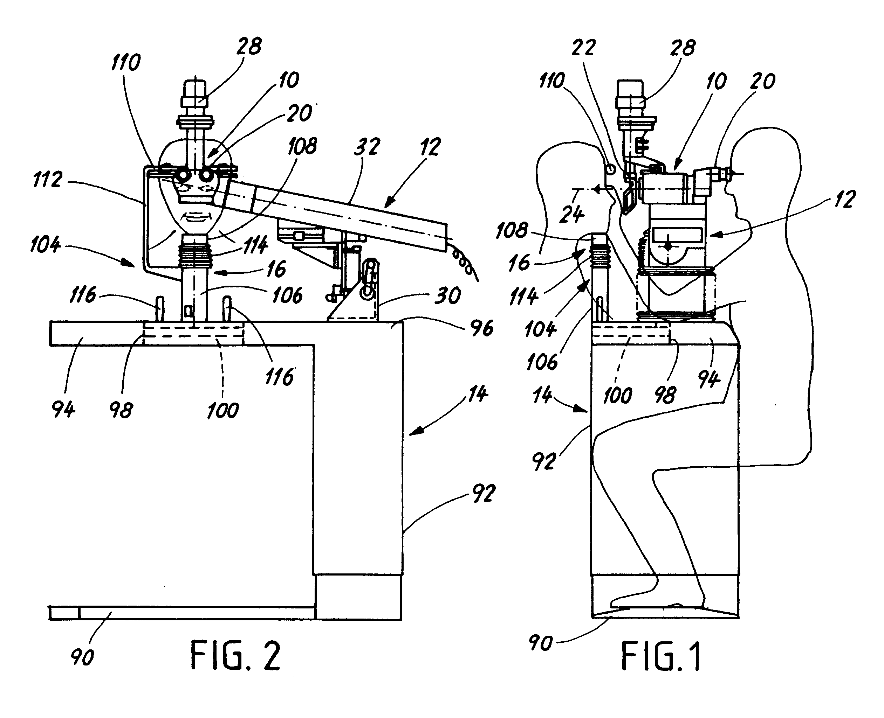

Les figures 1 et 2 représentent une vue de profil et de face respectivement de l'appareil selon l'invention. Il est utilisé sur un patient assis. Cet appareil comporte un dispositif d'observation et de traitement 10, un équipage 12 pour assurer le positionnement et le réglage du dispositif 10, un châssis 14 pour supporter le dispositif 10 et l'équipage 12 ainsi qu'un système d'appui-tête 16 pour caler et positionner la tête du patient.

Le dispositif d'observation et de traitement est plus spécialement destiné aux soins de l'oeil. Il comporte un binoculaire 20 et un objectif 22 définissant ensemble un système optique pour l'observation du patient par le médecin. Ce système optique comporte en outre des lentilles, miroirs et/ou prismes, et des moyens de focalisation non représentés au dessin. Le système optique définit un axe optique de sortie 24.

Le dispositif 10 comporte en outre des moyens d'éclairage de l'oeil, formés d'une lampe à fente 28, d'un miroir 29, ainsi que de lasers, non représentés, pour le traitement et pour l'observation de l'oeil du patient.

La lampe à fente est de forme allongée, placée entre le médecin et le patient, perpendiculairement à l'axe optique 24 et au-dessus de celui-ci lorsque le dispositif est adapté pour l'examen d'un patient assis. Placée ainsi, la lampe ne gêne pas le médecin dans son travail.

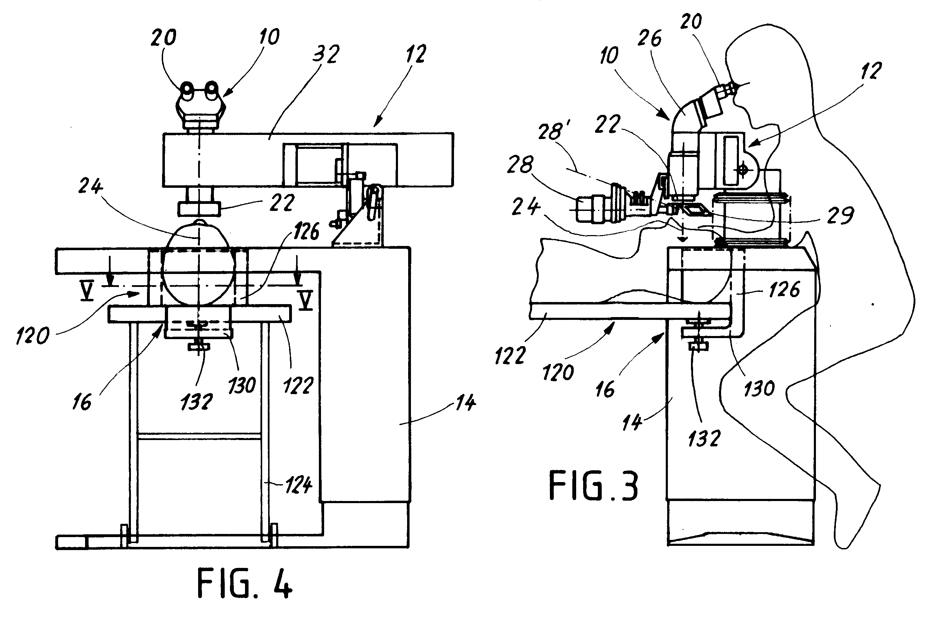

En variante, la lampe 28 peut être légèrement inclinée, comme représenté schématiquement par le trait mixte 28', visible sur la figure 3, de manière à l'éloigner du patient. La lampe est mobile et peut par exemple tourner sur un rail en arc de cercle, non représenté, et dont la concavité se trouve du côté du patient. Elle comporte des moyens de blocage non représentés, pour éviter un déplacement intempestif lorsque l'appareil est agencé pour traiter un patient en position couchée.

L'équipage, le châssis et le système d'appui-tête seront décrits ultérieurement plus en détail. Sur les figures 1 et 2, on peut d'ores et déjà remarquer que l'appareil permet l'observation et le traitement d'un patient en position assise. A cet effet, le patient a sa tête calée sur le système d'appui-tête, un oeil aligné sur l'axe de sortie 24 du dispositif 10. L'alignement de l'axe et de l'oeil est réalisé par le médecin, au moyen de dispositifs de commande non représentés sur ces figures.

Le médecin, assis en face du patient, observe l'oeil de ce dernier au travers du binoculaire 20 pour l'établissement d'un diagnostic, mais aussi pour activer le ou les lasers lorsqu'un traitement doit être effectué.

Nous nous référons maintenant aux figures 3 et 4, qui représentent le même appareil, agencé cette fois pour permettre l'observation et le traitement de l'oeil d'un patient en position couchée.

Sur ces figures, on retrouve le dispositif d'observation et de traitement 10, l'équipage 12 pour assurer le positionnement et le réglage du dispositif 10, le châssis 14 pour supporter le dispositif 10 et l'équipage 12, ainsi que le système d'appui-tête 16 pour caler et positionner la tête du patient. On peut voir également le binoculaire 20, l'objectif 22 définissant l'axe de sortie 24, ainsi que les moyens d'éclairage de l'oeil 28.

Le système optique comporte en outre un coude 26, comprenant un prisme non représenté au dessin. Le coude est amovible, par exemple fixé par enclenchement à baïonnette.

Avec l'appareil qui vient d'être décrit, le patient est couché, et l'axe optique 24 est vertical. Le médecin est assis, et observe l'oeil du patient au travers du binoculaire 20. Grâce à la présence du coude 26, le médecin peut rester assis pour observer et traiter le patient, le binoculaire 20 formant en effet un angle faible avec un plan horizontal.

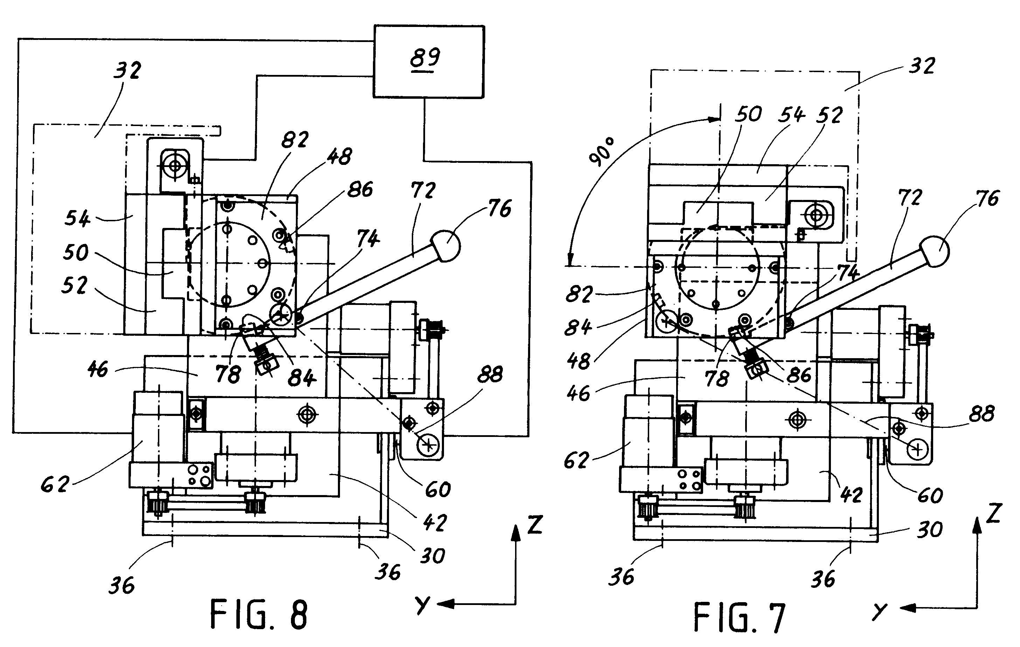

Nous décrirons maintenant plus en détail l'équipage 12, représenté aux figures 7 à 9. Il comporte un support d'équipage 30, solidaire du châssis 14, une armature 32 ainsi que des moyens de liaison de l'armature 32 au support 30, qui permettent de positionner et de déplacer l'armature 32 et par là le dispositif 10.

Plus précisément, le support 30 est fixé au châssis au moyen de vis schématiquement représentées en 36.

Ainsi qu'on peut plus particulièrement le voir sur les figures 2, 4 et 6, l'armature 32 est formée d'un tube portant le dispositif 10 à l'une de ses extrémités. Ce tube est sensiblement orienté horizontalement entre le patient et le médecin et va en se rétrécissant du support vers le dispositif. L'intérieur du tube est avantageusement utilisé pour y loger différents éléments associés au dispositif 10, par exemple un ou des lasers, ou encore des fibres optiques, des fils électriques, etc., reliant audit dispositif des appareils lourds disposés dans le châssis 14, voire même à côté de celui-ci.

Les moyens de liaison comprennent plus particulièrement une première partie pour permettre le déplacement du dispositif 10 selon quatre degrés de liberté et une seconde partie pour commander le mouvement et le positionnement du dispositif 10. La première partie comprend un ensemble de trois organes de guidage orthogonaux, comprenant chacun un rail et une coulisse, ainsi qu'un palier assurant une rotation autour d'un axe horizontal

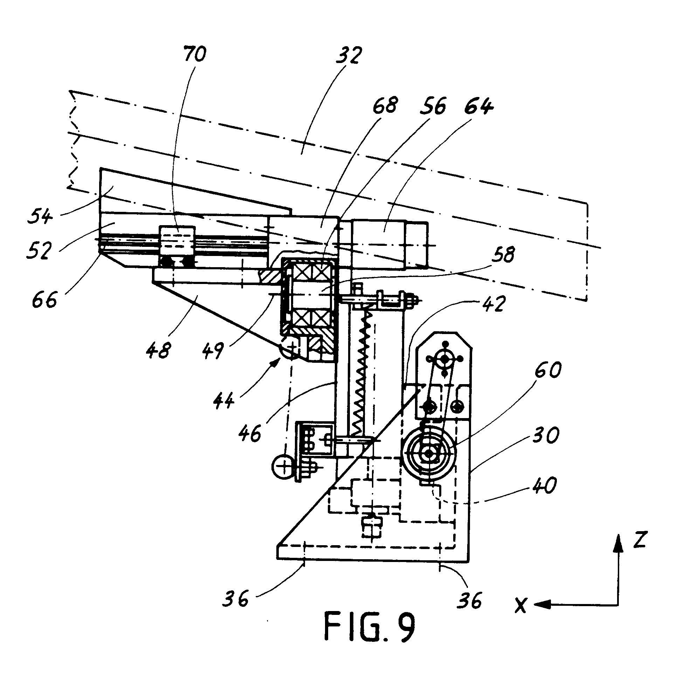

Un premier rail 40 (figure 9), orienté selon l'axe X du référentiel représenté aux figures 7 à 9, est solidaire du support 30. Ce rail 40 coopère avec un chariot 42, comportant deux coulisses orthogonales, dont l'une est engagée sur le rail 40. Cette première partie comporte en outre une potence 44, formée d'un rail d'axe Z engagé dans la seconde coulisse du chariot 42, d'un montant 46 fixé aux rail d'axe Z et d'une console 48, reliée au montant 46 de manière articulée, mobile en rotation autour d'un axe 49 parallèle à l'axe Y, par des moyens qui seront décrits ci-après.

La console 48 porte un rail 50 d'axe Y. Une coulisse 52, engagée sur le rail 50, porte l'armature 32 par l'intermédiaire d'un plateau 54.

L'articulation de la console 48 sur le montant 46 est réalisée au moyen d'un double roulement à billes 56 dont les cages sont fixées à la console 48, et dans lequel est engagé un arbre 58 solidaire du montant 46.

Dans cette configuration, l'équipage 12 comprend donc quatre degrés de liberté, soit trois en translation et un en rotation, l'axe de rotation étant horizontal.

La deuxième partie de l'équipage, destinée à commander le mouvement et le positionnement du dispositif 10, comprend trois ensembles identiques dans leur principe, entraînant l'armature chacun selon un axe. Chaque ensemble comporte un moteur, une vis sans fin, un écrou et une liaison desmodromique entre le moteur et la vis sans fin. Plus précisément, cette deuxième partie comprend un moteur 60 fixé sur le support 30. Ce moteur entraîne, par l'intermédiaire d'une liaison desmodromique, le chariot 42, notamment par un écrou solidaire de ce dernier, par une vis sans fin montée sur le support, et par un rouage et/ou une courroie crantée. Le rouage, la vis et l'écrou ne sont pas visibles au dessin.

Un moteur 62, fixé sur le chariot 42, entraîne selon l'axe Z le montant 46, par des moyens similaires à ceux associés au moteur 60.

Un moteur 64 (figure 9), monté sur la console 48, assure le déplacement selon l'axe Y. L'entraînement est réalisé au moyen d'une vis sans fin 66, par l'intermédiaire d'un rouage logé dans un boîtier 68. La vis sans fin 66 coopère avec un écrou 70 fixé au plateau 54.

Ainsi que cela a été expliqué ci-dessus, l'armature peut pivoter autour d'un axe horizontal. Il est bien clair que des manipulations intempestives doivent être empêchées. C'est pourquoi il est nécessaire de prévoir des moyens de blocage de l'armature dans ces positions extrêmes. A cet effet, l'équipage comporte un levier 72 (figures 7 et 8) qui pivote en 74 sur le montant 46. Le levier 72 comprend une poignée 76 à l'une de ses extrémités, et un doigt 78 à l'autre extrémité. Un ressort, non représenté au dessin, et dont une extrémité est fixée au montant 46 et l'autre au levier 72, tend à appliquer le levier 72 et plus spécialement son doigt 78 contre une came 82 solidaire de la console 48. Cette came 82 comporte une partie en arc de cercle, qui s'étend sur environ 90°, avec des encoches 84 et 86 à chacune de ses extrémités. Ces encoches 84 et 86 sont agencées de manière que le doigt 78 puisse s'y engager sans jeu.

Lorsque le médecin fait basculer l'armature, il est bien clair qu'il faut à tout prix éviter des chocs. C'est pourquoi l'équipage comporte en outre un frein hydraulique schématiquement représenté en 88, qui garantit un basculement de l'armature sans à-coup.

Pour commander le positionnement de l'armature, le médecin dispose d'un boîtier de commande, schématiquement représenté en 89 (figure 8), qui permet de commander l'entraînement en rotation des moteurs 60, 62 et 64. Ce boîtier de commande est décrit en détail dans une demande de brevet français intitulée "Dispositif de commande d'un appareil de traitement ophtalmologique" au nom de la Demanderesse, auquel on pourra se reporter.

Nous décrirons maintenant de manière plus complète le châssis 14, en référence aux figures 1 à 4 et 6.

Ce châssis comporte un socle 90, un montant 92 et une table 94. Le montant 92 est solidaire de l'une des extrémités du socle 90. Il a sensiblement la hauteur d'une table de travail classique, soit environ 80 cm. Il peut avantageusement comporter des moyens pour ajuster cette hauteur. La table 94 est fixée au montant 92, en porte-à-faux au-dessus du socle 90. La partie 96 de la table 94 se trouvant dans le prolongement du montant 92 porte le support 30.

La table 94 comporte, du côté destiné à recevoir le patient, une découpe 98, de forme trapézoïdale.

Lorsque l'appareil est agencé pour permettre l'observation et le traitement d'un patient assis tel que représenté aux figures 1 et 2, la découpe 98 est obturée par une pièce 100 de forme complémentaire, munie d'un coulisseau, engagé dans une coulisse pratiquée dans l'épaisseur de la table.

La pièce 100 porte un ensemble 104 permettant le positionnement de la tête d'un patient en position assise. Cet ensemble comprend un colonne 106 fixée à la pièce 100, une mentonnière 108 surmontant la colonne 106, un appui frontal 110, relié à la colonne 106 par une potence d'appui-tête 112. La colonne 106 est en outre munie de moyens permettant d'adapter la position de la mentonnière 108 en fonction de la morphologie du patient, schématiquement représentés par le soufflet 114. Ces moyens peuvent comporter un piston hydraulique par exemple.

Afin d'améliorer la stabilité du patient et de diminuer sa fatigue, l'ensemble 104 est complété par deux poignées 116 (figure 2), également solidaires de la pièce 100. Ces poignées, lorsqu'elles sont tenues par le patient, favorisent une position du torse et de la tête garantissant de bonnes conditions de travail.

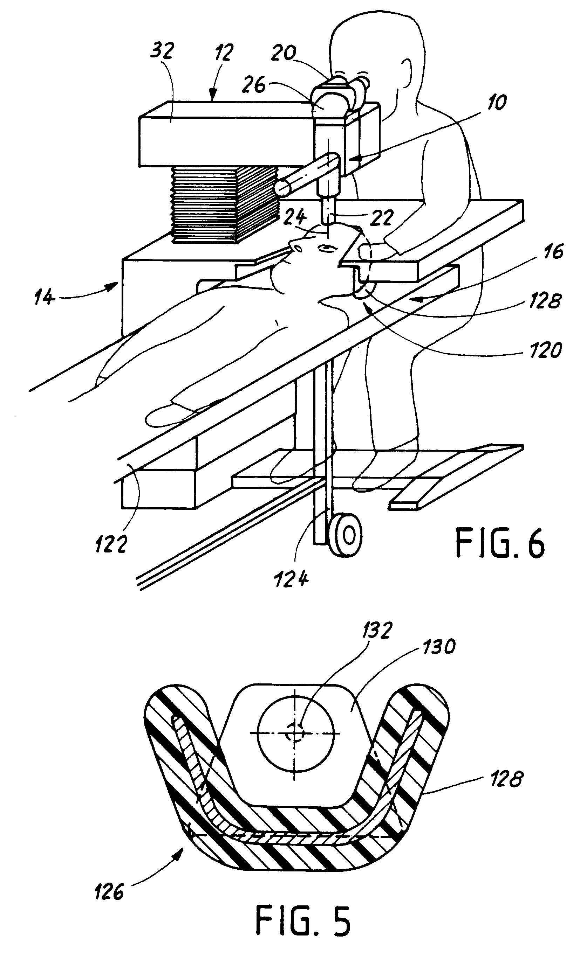

Sur les figures 3 à 6, on peut voir un ensemble 120 permettant notamment le positionnement de la tête d'un patient en position couchée. Il comprend un lit 122 muni de pieds 124 montés sur roulettes, et un cale-tête 126. Le cale-tête 126, plus particulièrement visible sur la figure 5, comporte une partie supérieure 128, en forme de U ouvert, et réalisée en un matériau rigide rembourré, et une équerre de fixation 130, munie d'une vis de blocage 132. Le cale-tête 126 est agencé de manière à être fixé solidement sur le lit 122. Sa partie supérieure 128 présente une forme extérieure complémentaire à celle de la découpe 98. Par ailleurs, l'ouverture du U est dimensionnée de manière que la tête du patient puisse y être calée et positionnée.

De la sorte, pour traiter un patient en position couchée, le cale-tête 126 est tout d'abord fixé sur la table 122. Le patient est ensuite installé, sa tête étant positionnée et calée dans le cale-tête 126. Le lit 122 est ensuite installé, en engageant le cale-tête 126 dans la découpe 98. Ainsi, la tête du patient est protégée lors de l'installation et est d'ores et déjà prépositionnée. Il ne reste donc plus au médecin qu'à ajuster la position du dispositif 10, grâce aux moyens de commande, et à adapter le dispositif à la morphologie particulière du patient, pour pouvoir observer et traiter l'un ou l'autre de ses yeux.

Ainsi que cela a été expliqué, un tel appareil permet donc d'observer et de traiter un patient tant en position assise que couchée, et cela pour une très faible augmentation du coût de l'appareil.

L'appareil tel que décrit est susceptible de comporter divers perfectionnements. Ainsi, tout ou partie de l'équipage peut avantageusement être protégé par un soufflet, comme on peut le voir aux figures 1, 3 et 6.

Le dispositif de commande 89 tel que prévu comporte un ensemble de touches, chaque touche étant associée à l'un des moteurs de l'équipage. En variante, il serait également possible de prévoir un détecteur de position de l'armature, permettant au dispositif de commande de savoir si l'appareil est en position de travail avec un patient assis ou couché. Dans ce cas, un organe de commande pourrait être associé à la commande du déplacement du dispositif dans la direction de l'oeil du patient. En position assise, cet organe de commande serait associé au moteur 60 d'axe X, alors qu'en position couchée, il serait associé au moteur 64 d'axe Z. Un deuxième organe de commande serait associé à la commande du déplacement du dispositif dans une direction perpendiculaire à l'axe reliant les deux yeux. En position assise, cet organe serait associé au moteur d'axe Z et, en position couchée, au moteur d'axe X. De la sorte, le médecin disposerait d'un dispositif de commande dont le comportement en regard du patient serait le même, que celui-ci soit couché ou assis.

L'agencement particulier des différents lasers que comporte l'appareil selon l'invention est décrit dans la demande de brevet français intitulée "Dispositif pour le traitement chirurgical d'un point situé dans un oeil", déposée par la Demanderesse en même temps que la présente demande. Il ne sera donc pas décrit en détail ici.

On relèvera toutefois que certains lasers de puissance ne peuvent pas être intégrés à l'appareil, à cause des très forts dégagements de chaleur.

Pour relier ces lasers de puissance au dispositif, on utilise des fibres optiques non représentées au dessin.

Une première fibre est fixée au laser, et une deuxième au dispositif, ces deux fibres étant reliées l'une à l'autre par un connecteur en deux parties, l'une fixée à l'extrémité de l'armature 32 voisine du support, l'autre à l'extrémité libre de la première fibre.

Grâce à cette configuration particulière, la liaison entre le ou les lasers de puissance et le dispositif est assurée bien qu'elle se fasse par l'intermédiaire d'une armature mobile.

## Revendications

1. Appareil pour l'observation et le traitement d'un patient, qui comporte
- un dispositif (10) d'observation et de traitement, définissant un axe optique de sortie (24),
- un équipage (12) pour assurer le positionnement et le réglage dudit dispositif (10),
- un châssis (14) pour supporter ledit équipage (12) et ledit dispositif (10), et
- un système d'appui-tête (16) pour caler et positionner la tête du patient,
caractérisé en ce que ledit équipage (12) comprend un support (30) monté rigidement sur le châssis (14), une armature (32) solidaire dudit dispositif (10), et une articulation (56) qui relie ledit support (30) et ladite armature (32), agencée pour permettre une rotation de l'armature (32) autour d'un axe horizontal, de manière à assurer une orientation angulaire dudit axe optique (24) selon deux directions permettant respectivement l'observation et/ou le traitement d'un patient en positions assise et couchée.

2. Appareil selon la revendication 1, caractérisé en ce que ledit équipage (12) comporte en outre un ensemble de rails (40, 44, 50) et de coulisses (42, 52) coopérant deux à deux, pour permettre un déplacement de l'armature (32) par rapport au châssis (14), selon trois directions orthogonales.

3. Appareil selon les revendications 1 ou 2, caractérisé en ce que ladite armature (32) comporte un bras en porte-à-faux, fixé au châssis par l'intermédiaire dudit équipage (12) et portant ledit dispositif (10) à son extrémité libre.

4. Appareil selon l'une quelconque des revendications 1 à 3, caractérisé en ce que ledit dispositif (10) est destiné à l'observation et au traitement d'un oeil du patient.

5. Appareil selon l'une quelconque des revendications 1 à 4, caractérisé en ce que ledit système d'appui-tête (16) comporte un premier ensemble (104) susceptible d'être fixé de manière amovible sur le châssis, pour caler et positionner la tête d'un patient assis, et un second ensemble (126), associé à un lit (122) destiné à recevoir un patient couché, pour caler et positionner la tête de celui-ci.

6. Appareil selon la revendication 5, caractérisé en ce que le châssis (14) comprend une table (94) munie d'une découpe (98) et une pièce (100) amovible de forme complémentaire engagée et positionnée dans la découpe (98) et portant ledit premier ensemble (104).

7. Appareil selon les revendications 5 ou 6, caractérisé en ce que le second ensemble (126) comporte un bloc de positionnement définissant une cavité adaptée à la morphologie de la tête du patient pour maintenir celle-ci en position fixe par rapport au châssis (14) lorsque l'appareil est adapté pour l'observation et le traitement en position couchée.

8. Appareil selon les revendications 6 et 7, caractérisé en ce que la forme extérieure dudit bloc (126) est complémentaire à celle de ladite découpe (98) et en ce qu'il est engagé et positionné dans la découpe (98).

9. Appareil selon l'une quelconque des revendications 1 à 8, caractérisé en ce que ledit dispositif comporte un objectif (22) se trouvant au voisinage de l'oeil du patient, un oculaire (20) pour l'observation de l'oeil du patient par un médecin et un organe d'infléchissement (26) du faisceau d'observation, pour permettre au médecin d'être assis lorsqu'il observe et traite un patient couché.

## Claims

1. Apparatus for observing and treating a patient which includes
- an observing and treating arrangement (10) defining an optical output axis (24),
- means (12) for effecting positioning and adjustment of said arrangement (10),
- an underframe (14) for supporting said means (12) and said arrangement (10), and
- a head rest system (16) for immobilizing and positioning the head of the patient,
characterized in that said means (12) comprises a support (30) rigidly mounted on the underframe (14) , an armature (32) integral with said arrangement (10) and a link (56) which couples said support (30) and said armature (32) arranged to permit rotation of the armature (32) around a horizontal axis in order to ensure an angular orientation of said optical axis (24) in two directions so as to permit observation and/or treatment of a patient in a seated and recumbent position respectively.

2. Apparatus according to claim 1, characterized in that said means (12) further includes an assembly of rails (40, 44, 50) and slideways (42, 52) cooperating two by two thereby to enable displacement of the armature (32) relative to the underframe (14) in three orthogonal directions.

3. Apparatus according to claims 1 or 2, characterized in that said armature (32) includes a cantilevered arm fastened to the underframe through said means (12) and bearing said arrangement (10) at its free end.

4. Apparatus according to claims 1 to 3, characterized in that said arrangement (10) is adapted for the observation and treatment of an eye of the patient.

5. Apparatus according to any one of claims 1 to 4, characterized in that said head rest system (16) includes a first assembly (104) adapted to be detachably fastened to the underframe in order to immobilize and position the head of a seated patient and a second assembly (126) associated with a bed (122) intended for a recumbent patient so as to immobilize and position the head thereof.

6. Apparatus acording to claim 5, characterized in that the underframe (14) comprises a table (94) provided with a cutout (98) and a removable piece (100) of complementary form accommodated and positioned in the cutout (98) and bearing said first assembly (104).

7. Apparatus according to claims 5 or 6, characterized in that the second assembly (126) includes a positioning block defining a cavity adapted to the form of the patient's head so as to maintain the latter in a fixed position relative to the underframe (14) when the apparatus is adapted for observation and treatment of a recumbent patient.

8. Apparatus according to claims 6 and 7, characterized in that the outer form of said block (126) is complementary to that of said cutout (98) and in that it may be accommodated and positioned in said cutout (98).

9. Apparatus according to any one of claims 1 to 8, characterized in that said arrangement includes a lens (22) located proximate the eye of the patient, an eyepiece (20) for enabling observation of the eye of the patient by a medical practitioner and means (26) for deviating the observation beam whereby the practitioner may be seated when observing and treating a recumbent patient.

## Patentansprüche

1. Apparat für die Untersuchung und die Behandlung eines Patienten, welcher Apparat umfaßt,
- eine Untersuchungs- und Behandlungsvorrichtung (10), welche eine optische Ausgangsachse (24) definiert,
- eine Einrichtung (12) zum Sicherstellen der Positionierung und der Einregulierung der Vorrichtung (10),
- ein Chassis (14) zum Abstützen der Einrichtung (12) und der Vorrichtung (10), und
- ein Kopfanschlagsystem (16) zum Immobilisieren und Positionieren des Kopfes des Patienten,
dadurch gekennzeichnet, daß die Einrichtung (12) einen Support (30) umfaßt, der starr an dem Chassis (14) montiert ist, eine Armatur (32), die mit der Vorrichtung (10) verbunden ist, und eine Gelenkanordnung (56), die den Support (30) und die Armatur (32) verbindet und ausgebildet ist zum Ermöglichen der Drehung der Armatur (32) um eine horizontale Achse derart, daß eine Winkelorientierung der optischen Achse (24) in zwei Richtungen sichergestellt wird, die die Untersuchung und/oder die Behandlung eines Patienten in sitzender beziehungsweise liegender Position ermöglichen.

2. Apparat nach Anspruch 1, dadurch gekennzeichnet, daß die Einrichtung (12) ferner eine Baugruppe aus paarweise zusammenwirkenden Schienen (40, 44, 50) und Führungen (42, 52) umfaßt, um eine Verlagerung der Armatur (32) relativ zum Chassis (14) in drei orthogonalen Richtungen zu ermöglichen.

3. Apparat nach Anspruch 1 oder 2, dadurch gekennzeichnet, daß die Armatur (32) einen freitragenden Arm umfaßt, der an dem Chassis über die Einrichtung (12) befestigt ist und die Vorrichtung (10) an seinem freien Ende trägt.

4. Apparat nach einem der Ansprüche 1 bis 3, dadurch gekennzeichnet, daß die Vorrichtung (10) zur Untersuchung und Behandlung des Auges des Patienten bestimmt ist.

5. Apparat nach einem der Ansprüche 1 bis 4, dadurch gekennzeichnet, daß das Kopfanschlagsystem (16) eine erste Baugruppe (104) umfaßt, die an dem Chassis lösbar befestigbar ist zum Immobilisieren und Positionieren des Kopfes eines sitzenden Patienten, sowie eine zweite Baugruppe (126), zugeordnet einer Liege (122), bestimmt zur Aufnahme eines liegenden Patienten zum Immobilisieren und Positionieren des Kopfes des letzteren.

6. Apparat nach Anspruch 5, dadurch gekennzeichnet, daß das Chassis (14) einen Tisch (94) umfaßt, versehen mit einem Ausschnitt (98), und ein herausnehmbares Bauteil (100) in komplementärer Form, eingefügt und positioniert in dem Ausschnitt (98), welches die erste Baugruppe (104) trägt.

7. Apparat nach Anspruch 5 oder 6, dadurch gekennzeichnet, daß die zweite Baugruppe (126) einen Positionierblock umfaßt, der einen an die Morphologie des Kopfes des Patienten angepaßten Hohlraum definiert zum Halten desselben in fester Position relativ zum Chassis (14), wenn der Apparat für die Untersuchung und Behandlung in liegender Position eingerichtet ist.

8. Apparat nach Anspruch 6 und 7, dadurch gekennzeichnet, daß die äußere Form des Blocks (126) komplementär ist zu der des Ausschnitts (98) und daß er in dem Ausschnitt (98) eingefügt und positioniert ist.

9. Apparat nach einem der Ansprüche 1 bis 8, dadurch gekennzeichnet, daß die Vorrichtung ein Objektiv (22) umfaßt, das sich nahe dem Auge des Patienten befindet, ein Okular (20) für die Untersuchung des Auges des Patienten durch einen Mediziner, und ein Ablenkorgan (26) für das Untersuchungslichtbündel umfaßt, um dem Mediziner zu ermöglichen, sitzend einen liegenden Patienten zu untersuchen und zu behandeln.
